# EUROPEAN PATENT APPLICATION

(11) **EP 4 238 478 A1**
(43) Date of publication of application: **06.09.2023**
(21) Application number: 20958988.6
(22) Date of filing: 27.10.2020
(51) Int. Cl.: A61B 1/05

(54) **ENDOSCOPE PHOTOGRAPHING SYSTEM AND IMAGE DATA TRANSMISSION APPARATUS THEREFOR**

(71) Applicant: Shenzhen Mindray Bio-Medical Electronics Co., Ltd., Shenzhen, Guangdong 518057 (CN)
(72) Inventor: XU, Tao, Shenzhen, Guangdong 518057 (CN); WEI, Kaiyun, Shenzhen, Guangdong 518057 (CN)
(74) Representative: KIPA AB
(86) International application number: PCT/CN2020/123991
(87) International publication number: WO 2022/087823

(57) **Abstract**

An image data transmission apparatus for an endoscope photographing system, configured to be connected to an image processing portion (500) of the endoscope photographing system so as to transmit image data to the image processing portion (500), and comprising: an image sensor (10, 11, 12) used for generating and outputting image data based on a first data communication protocol; a first data processing device (20) comprising a programmable logic gate array device, the first data processing device (20) being at least used for converting the image data outputted by the image sensor (10, 11, 12) to image data based on a second data communication protocol and outputting same, and the second data communication protocol being different from the first data communication protocol; and an optical fiber transmission assembly (30) used for converting the image data based on the second data communication protocol outputted by the first data processing device (20) from an electrical signal to an optical signal for transmission, and then converting the optical signal to an electrical signal and outputting same for the image processing portion (500) to process so as to generate data for displaying an image.

## Description

### TECHNICAL FIELD

The disclosure relates to an endoscope imaging system and image data transmission apparatus therefor.

### BACKGROUND

In recent years, endoscopic imaging systems have been increasingly widely used in surgical procedures and diagnostic examinations. The endoscopic imaging system can provide interior images of human body to a doctor, who can stably and correctly perform surgery or examination through the images.

Resolution of endoscopic imaging system has gone through a development process of high-definition (HD), full high-definition (FHD), and then ultra-high definition (UHD), or in other words, a development process of 1K, 2K, and then 4K. While the resolution continues to improve, there are also many technical issues that need to be addressed.

For example, with the continuous improvement of the resolution of the endoscopic imaging system, its sensor for data acquisition acquires an increasing amount of image data. The acquired data should be transmitted to a host of the endoscopic imaging system for processing through a transmission cable component. In order to ensure real-time performance and display efficiency, the transmission cable component needs to be able to transmit a large amount of data at a high speed and have a good anti-interference ability.

At present, image data transmission from the sensor to the image processing host in endoscope imaging systems is generally achieved through multi-channel twisted pair cables. This has many drawbacks, such as the overall cable being too thick, poor anti-interference ability, and usually being unable to satisfy real-time transmission of high-resolution image data.

### SUMMARY

An image data transmission apparatus for an endoscope imaging system is provided, wherein the image data transmission apparatus is connected with an image processing unit in the endoscope imaging system and is configured to transmit image data to the image processing unit; wherein the image data transmission apparatus includes:
at least a first image sensor and a second image sensor; wherein the first image sensor and the second image sensor are both configured to generate image data, and the first image sensor and the second image sensor are configured to output the image data based on a first data communication protocol through their respective data output channel;
a first data processing device which includes a programmable logic gate array device; wherein the programmable logic gate array device includes at least a first group of data output terminals and a second group of data output terminals; wherein the first data processing device is configured to convert the image data which is outputted by the first image sensor to a first set of image data based on a second data communication protocol, and output the first set of image data through the first group of data output terminals, as well as to convert the image data which is outputted by the second image sensor to a second set of image data based on the second data communication protocol, and output the second set of image data through the second group of data output terminals; wherein the second data communication protocol is different from the first data communication protocol;
an optical fiber transmission component, which includes an electro-optic converter, a photo-electric converter, at least a first optical fiber transmission channel, and a second optical fiber transmission channel; wherein the electro-optic converter is configured to convert the first set of image data which is outputted by the first data processing device from an electrical signal to an optical signal, and transmit the converted first set of image data to the photo-electric converter through the first optical fiber transmission channel; and the photo-electric converter is configured to convert the received first set of image data from the optical signal to an electrical signal and output the further converted first set of image data; wherein the electro-optic converter is further configured to convert the second set of image data which is outputted by the first data processing device from an electrical signal to an optical signal, and transmit the converted second set of image data to the photo-electric converter through the second optical fiber transmission channel, and the photo-electric converter is configured to convert the received second set of image data from the optical signal to an electrical signal and output the further converted second set of image data; wherein the further converted first set of image data and the further converted second set of image data which are outputted by the photo-electric converter are processed by the image processing unit to generate data for image displaying.

In an embodiment, the respective data output channel of the first image sensor and the second image sensor is an MIPI CSI interface, and the first data communication protocol is an MIPI CSI protocol.

In an embodiment, the second data communication protocol satisfies a requirement that the image data based on the second data communication protocol have a signal amplitude which is required for converting the image data from the electrical signal to the optical signal by the electro-optic converter.

In an embodiment, the second data communication protocol is capable of being directly identified by the image processing unit.

In an embodiment, the image data transmission apparatus further includes a first bridging circuit which is connected between the first image sensor and the first data processing device, and/or a second bridging circuit which is connected between the second image sensor and the first data processing device.

In an embodiment, a data output channel of the first bridging circuit has a lower data transmission rate than the data output channel of the first image sensor, and a data output channel of the second bridging circuit has a lower data transmission rate than the data output channel of the second image sensor.

In an embodiment, the first bridging circuit includes more data output channels than the first image sensor, and the second bridging circuit includes more data output channels than the second image sensor.

In an embodiment, the first bridging circuit and the second bridging circuit are configured to convert the image data based on the first data communication protocol to image data based on a third data communication protocol; wherein the third data communication protocol is different from the first data communication protocol and the second data communication protocol, wherein the third data communication protocol has a lower data transmission rate than the first data communication protocol.

In an embodiment, the image data transmission apparatus further includes a third bridging circuit and/or a fourth bridging circuit which are/is connected between the optical fiber transmission component and the image processing unit; the third bridging circuit is configured to convert the first set of image data based on the second data communication protocol to the image data based on a fourth data communication protocol, and the fourth bridging circuit is configured to convert the second set of image data based on the second data communication protocol to the image data based on a fourth data communication protocol; wherein the fourth data communication protocol is different from the first data communication protocol and the second data communication protocol, wherein the fourth data communication protocol has a lower data transmission rate than the second data communication protocol.

In an embodiment, the first optical fiber transmission channel includes a first optical fiber, and the second optical fiber transmission channel includes a second optical fiber;
the electro-optic converter includes at least a first input terminal, a second input terminal, a first output terminal, and a second output terminal; the photo-electric converter includes at least a first input terminal, a second input terminal, a first output terminal, and a second output terminal; wherein the first output terminal of the electro-optic converter is connected with the first input terminal of the photo-electric converter through the first optical fiber; the second output terminal of the electro-optic converter is connected with the second input terminal of the photo-electric converter through the second optical fiber;
the electro-optic converter is configured to receive the first set of image data which is outputted by the first data processing device through the first input terminal of the electro-optic converter, and to convert the first set of image data from the electrical signal to the optical signal; the electro-optic converter is further configured to output the first set of image data, which is converted to the optical signal, through the first output terminal of the electro-optic converter, and to transmit converted the first set of image data through the first optical fiber; the photo-electric converter is configured to receive the first set of image data which is converted to the optical signal and transmitted by the first optical fiber through the first input terminal of the photo-electric converter, to convert the converted first set of image data from the optical signal to the electrical signal, and to output the further converted first set of image data through the first output terminal of the photo-electric converter;
the electro-optic converter is configured to receive the second set of image data which is outputted by the first data processing device through the second input terminal of the electro-optic converter, and to convert the second set of image data from the electrical signal to the optical signal; the electro-optic converter is further configured to output the second set of image data, which is converted to the optical signal, through the second output terminal of the electro-optic converter, and to transmit the converted second set of image data through the second optical fiber; the photo-electric converter is configured to receive the second set of image data which is converted to the optical signal and transmitted by the second optical fiber through the second input terminal of the photo-electric converter, to convert the converted second set of image data from the optical signal to the electrical signal, and to output the further converted second set of image data through the second output terminal of the photo-electric converter.

In an embodiment, the electro-optic converter includes a first electro-optic converter and a second electro-optic converter; the photo-electric converter includes a first photo-electric converter and a second photo-electric converter; the first optical fiber transmission channel includes a first optical fiber, and the second optical fiber transmission channel includes a second optical fiber;
the first electro-optic converter includes a first input terminal and a first output terminal, the second electro-optic converter includes a second input terminal and a second output terminal, the first photo-electric converter includes a first input terminal and a first output terminal, the second photo-electric converter includes a second input terminal and a second output terminal; wherein the first output terminal of the first electro-optic converter is connected with the first input terminal of the first photo-electric converter through the first optical fiber; the second output terminal of the second electro-optic converter is connected with the second input terminal of the second photo-electric converter through the second optical fiber;
the first electro-optic converter is configured to receive the first set of image data which is outputted by the first data processing device through the first input terminal of the first electro-optic converter, and to convert the first set of image data from the electrical signal to the optical signal; the first electro-optic converter is further configured to output the first set of image data which is converted to the optical signal through the first output terminal of the first electro-optic converter, and to transmit the converted first set of image data through the first optical fiber; the first photo-electric converter is configured to receive the first set of image data which is converted to the optical signal and transmitted by the first optical fiber through the first input terminal of the first photo-electric converter, to convert the converted first set of image data from the optical signal to the electrical signal, and to output the further converted first set of image data through the first output terminal of the first photo-electric converter;
the second electro-optic converter is configured to receive the second set of image data which is outputted by the first data processing device through the second input terminal of the second electro-optic converter, and to convert the second set of image data from the electrical signal to the optical signal; the second electro-optic converter is further configured to output the second set of image data which is converted to the optical signal through the second output terminal of the second electro-optic converter, and to transmit the converted second set of image data through the second optical fiber; the second photo-electric converter is configured to receive the second set of image data which is converted to the optical signal and transmitted by the second optical fiber through the second input terminal of the second photo-electric converter, to convert the converted second set of image data from the optical signal to the electrical signal, and to output the further converted second set of image data through the second output terminal of the second photo-electric converter.

An image data transmission apparatus for an endoscope imaging system is provided, wherein the image data transmission apparatus is connected with an image processing unit in the endoscope imaging system and is configured to transmit image data to the image processing unit; wherein the image data transmission apparatus includes:
an image sensor which is configured to generate and output image data based on a first data communication protocol;
a first data processing device which includes a programmable logic gate array device; wherein the first data processing device is configured to at least convert the image data, which is outputted by the image sensor, to image data based on a second data communication protocol, and to output the image data based on the second data communication protocol; wherein the second data communication protocol is different from the first data communication protocol;
an optical fiber transmission component, which is configured to convert the image data based on the second data communication protocol, which data is outputted by the first data processing device, from an electrical signal to an optical signal; and to transmit the image data which is converted to the optical signal; and then to convert the image data, which is converted to the optical signal, to an electrical signal; and to output the image data, which is converted to the electrical signal to the image processing unit for processing, so as to generate data for image displaying.

In an embodiment, a data output channel of the image sensor is an MIPI CSI interface, and the first data communication protocol is an MIPI CSI protocol.

In an embodiment, the second data communication protocol satisfies a requirement that the image data based on the second data communication protocol have a signal amplitude which is required for converting the image data from the electrical signal to the optical signal by the optical fiber transmission component.

In an embodiment, the second data communication protocol is capable of being directly identified by the image processing unit.

In an embodiment, the image data transmission apparatus further includes a first bridging circuit which is connected between the image sensor and the first data processing device.

In an embodiment, a data output channel of the first bridging circuit has a lower data transmission rate than a data output channel of the image sensor.

In an embodiment, the first bridging circuit includes more data output channels than the image sensor.

In an embodiment, the first bridging circuit is configured to convert the image data based on the first data communication protocol to image data based on a third data communication protocol; the third data communication protocol is different from the first data communication protocol and the second data communication protocol; wherein the third data communication protocol has a lower data transmission rate than the first data communication protocol.

In an embodiment, the image data transmission apparatus further includes a third bridging circuit which is connected between the optical fiber transmission component and the image processing unit, wherein the third bridging circuit is configured to convert the image data based on the second data communication protocol to image data based on a fourth data communication protocol; wherein the fourth data communication protocol is different from the first data communication protocol and the second data communication protocol, and the fourth data communication protocol has a lower data transmission rate than the second data communication protocol.

In an embodiment, the optical fiber transmission component includes an electro-optic converter, an optical fiber transmission channel, and a photo-electric converter;
the electro-optic converter is configured to receive the image data which is outputted by the first data processing device, and convert the image data from the electrical signal to the optical signal for outputting to the optical fiber transmission channel;
the optical fiber transmission channel is configured to transmit the image data which is converted to the optical signal, wherein the optical fiber transmission channel includes an optical fiber;
the photo-electric converter is configured to receive the image data which is converted to the optical signal and transmitted through the optical fiber transmission channel, to convert converted the image data from the optical signal to the electrical signal, and to output the image data which is further converted to the electrical signal.

An endoscope imaging system, is provided, including:
a light source unit;
a light source control unit, which is configured to control the light source unit to provide imaging light;
an endoscope, which includes an insertion portion which is insertable into an interior of an object;
a imaging unit, which includes the image data transmission apparatus according to any of the above embodiments;
an image processing unit, which is configured to receive and analyze the image data which is outputted by the image data transmission apparatus to generate the data for image displaying; and
a display, which is configured to display the data for image displaying.

In an embodiment, the image processor includes the programmable logic gate array or a central processor.

### BRIEF DESCRIPTION OF THE DRAWINGS

FIG. 1 is a structural diagram of an image data transmission apparatus in an embodiment of this disclosure.
FIG. 2 is a structural diagram of an image data transmission apparatus in an embodiment of this disclosure.
FIG. 3 is a structural diagram of an image data transmission apparatus in an embodiment of this disclosure.
FIG. 4 is a structural diagram of an image data transmission apparatus in an embodiment of this disclosure.
FIG. 5 is a structural diagram of an image data transmission apparatus in an embodiment of this disclosure.
FIG. 6 is a structural diagram of an image data transmission apparatus in an embodiment of this disclosure.
FIG. 7 is a structural diagram of an image data transmission apparatus in an embodiment of this disclosure.
FIG. 8 is a structural diagram of an image data transmission apparatus in an embodiment of this disclosure.
FIG. 9 is a structural diagram of an image data transmission apparatus in an embodiment of this disclosure.
FIG. 10 is a structural diagram of an image data transmission apparatus in an embodiment of this disclosure.
FIG. 11 is a structural diagram of an endoscope imaging system in an embodiment of this disclosure.
FIG. 12 is a structural diagram of an endoscope imaging system in an embodiment of this disclosure.
FIG. 13 is a structural diagram of an endoscope imaging system in an embodiment of this disclosure.
FIG. 14 is a structural diagram of an endoscope imaging system in an embodiment of this disclosure.
FIG. 15 is a structural diagram of an endoscope imaging system in an embodiment of this disclosure.
FIG. 16 is a structural diagram of an endoscope imaging system in an embodiment of this disclosure.
FIG. 17 is a structural diagram of an endoscope imaging system in an embodiment of this disclosure.
FIG. 18 is a structural diagram of an endoscope imaging system in an embodiment of this disclosure.
FIG. 19 is a structural diagram of an endoscope imaging system in an embodiment of this disclosure.
FIG. 20 is a structural diagram of an endoscope imaging system in an embodiment of this disclosure.
FIG. 21 is a structural diagram of an endoscope imaging system in an embodiment of this disclosure.
FIG. 22 is a structural diagram of an endoscope imaging system in an embodiment of this disclosure.
FIG. 23 is a structural diagram of an endoscope imaging system in an embodiment of this disclosure.
FIG. 24 is a structural diagram of an endoscope imaging system in an embodiment of this disclosure.
FIG. 25 is a structural diagram of an endoscope imaging system in an embodiment of this disclosure.

### DESCRIPTION OF THE PREFERRED EMBODIMENTS

This disclosure is further described in detail below through specific embodiments in combination with the accompanying drawings. In different embodiments, similar elements adopt associated similar reference numbers. In the following embodiments, many details are described in order to make this disclosure better understood. However, one skilled in the art can easily recognize that some of the features can be omitted in different cases, or can be replaced by other elements, materials and methods. In some cases, some operations related to this disclosure are not shown or described in the description, in order to avoid the core part of this disclosure being inundated by too many descriptions. For one skilled in the art, it is not necessary to describe these related operations in detail, as they can fully understand the relevant operations according to the description in this disclosure and the general technical knowledge in the art.

In addition, the features, operations, or characteristics described in this disclosure may be combined in any appropriate manner to form various embodiments. At the same time, the steps or actions in the method description can also be exchanged or adjusted in order in a manner obvious to one skilled in the art. Therefore, the various sequences in the description and the drawings are only for the purpose of clearly describing a certain embodiment, and do not mean that they are necessary sequences. Unless it is otherwise specified that one of the sequences must be followed.

The terms "first", "second", etc., in this disclosure are operable to distinguish different objects, rather than to describe a specific order. In addition, the terms "connection", and "linkage" mentioned in this disclosure include direct and indirect connection (linkage), unless otherwise specified.

As mentioned above, the current image data acquisition and transmission schemes for endoscope imaging system are difficult to satisfy the high-definition or even 4K requirements of real-time imaging.

Firstly, the current 4K image sensor are generally an image sensor with sub-LVDS interface, rather than an image sensor with MIPI CSI interface which has better performance. This is because the image acquisition component (camera head/camera handle) of the endoscope imaging system is constrained by size and power consumption, while the image sensor with MIPI CSI interface, having better performance, typically has four data channels, which enable a higher rate for a single channel, typically exceeding 1.5Gbps. However, the image sensor with sub-LVDS interface typically has eight to ten data channels. Accordingly, in the situation of same number, the rate of the single channel for the image sensor with sub-LVDS interface can be greatly reduced, for example, less than 1 Gbps. Therefore, the current endoscope imaging system cannot directly select such as the image sensor with MIPI CSI interface for image acquisition.

Secondly, the amount of the data which is acquired and transmitted by the current 4K image sensor is extremely large. In order to ensure the real-time performance, it is necessary to transmit the image data acquired by the image sensor to the image processing host at a high speed. However, the current image data transmission achieved through multi-channel twisted pair cables cannot satisfy this high-speed real-time transmission, and their anti-interference ability is poor, and they are thick in size.

Considering the high resolution, such as the 4K endoscope imaging system, there are still many technical problems to be solved. The applicant has studied one or more of these technical problems and proposed some solutions, which are explained in detail below.

In some embodiments, an image data transmission apparatus is provided, and the image data transmission apparatus in this disclosure can be applied to occasions and products such as endoscope imaging systems. The image data transmission apparatus of this disclosure can be connected with an image processing unit in an endoscope imaging system, such as an image processing host, to transmit image data to the image processing unit, and the image data transmitted to the image processing unit can be processed by the image processing unit to generate data for image displaying.

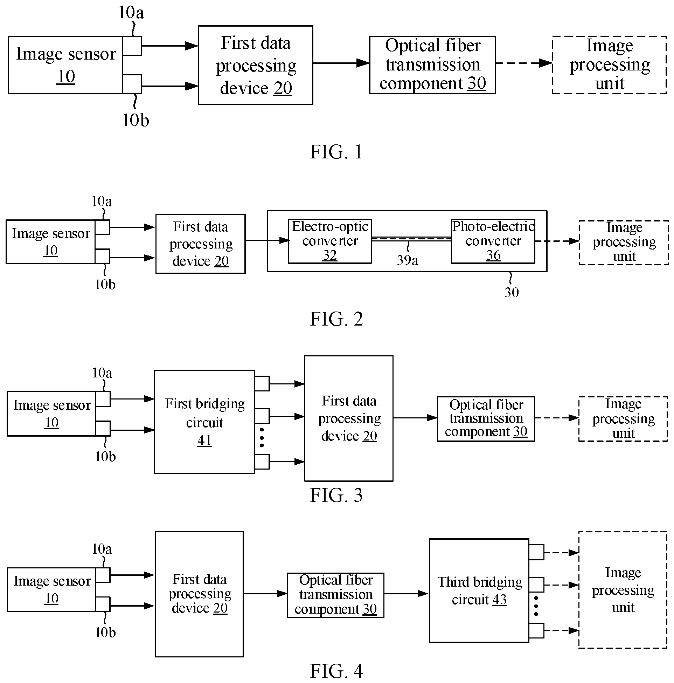

Please refer to FIG. 1. In some embodiments, the image data transmission apparatus includes an image sensor 10, a first data processing device 20, and an optical fiber transmission component 30, as explained in detail below.

The image sensor 10 is configured to generate and output image data based on a first data communication protocol. In some embodiments, the image sensor 10 includes at least two data output channels, such as data output channel 10a and data output channel 10b, through which the image sensor outputs the image data. The image sensor 10 can generate the image data according to specifications that can be processed by an AP (Application Processor) of a CPU for a mobile device.

The first data processing device 20 includes a programmable logic gate array device for converting the image data which is outputted by the image sensor 10 to image data based on a second data communication protocol and outputting said image data. Among them, the second data communication protocol is different from the first data communication protocol.

The optical fiber transmission component 30 is configured to convert the image data based on the second data communication protocol, which is outputted by the first data processing device 20, from an electrical signal to an optical signal for transmission, and then to convert the optical signal to the electrical signal and output it for processing by the image processing unit to generate data for image displaying. The optical fiber transmission component 30 is just used as a transparent channel and not involved in protocol packaging and unpacking work.

In an embodiment, the image sensor 10 can generate image data of MIPI (Mobile Industry Processor Interface) specification. For example, a data output channel of the image sensor is the MIPI CSI interface, and the first data communication protocol is the MIPI CSI protocol. Specifically, the data output channel of the image sensor can be the MIPI CSI-2 interface. MIPI is an alliance established in 2003 by companies such as ARM in the UK, Nokia in Finland, ST in Italy, and TI in the US, aiming at standardizing internal interfaces of mobile phones, such as camera, display interface, RF/baseband interface, etc. for reducing the complexity of mobile phone design and increase design flexibility. There are different working groups under the MIPI Alliance, which have defined a series of internal interface standards for mobile phones, such as camera serial interface (CSI), display serial interface (DSI), RF interface DigRF, microphone/speaker interface SLIMbus, etc. The terminal market requires lower power consumption, higher data transmission rate, and smaller PCB footprint. Among the several standard-based serial differential interfaces currently used, the MIPI interface is suitable for use in the field of power sensitive but high-performance devices. Specifically, as mentioned above, CSI is an interface standard designated by the Camera Working Group of MIPI Alliance, CSI-2 is the second version of MIPI CSI, mainly composed of an application layer, a protocol layer, and a physical layer. It usually supports four-channel data transmission, with a single line transmission speed of up to 1 Gb/s, and also supports eight-channel data transmission. In addition to the ground wire, the MIPI CSI-2 interface generally has one pair of I2C communication pins, one pair of MIPI differential clock pins, and one to four pairs of MIPI differential data signal pins.

Usually, the camera head of the endoscope imaging system is handheld, and the doctor holds the camera head to adjust observed portion and control parameters when performing a surgery on a patient. Due to the large amount of data transmitted by the image sensor, the transmission power is high, while significant heat is also generated. The camera head need to be designed with features such as low power consumption and minimal heat generation. The image sensor with MIPI interface has the characteristic of low power consumption, which precisely satisfies the design requirement of the camera head.

Therefore, in order to reduce the power consumption of related devices, this embodiment adopts an image sensor with a MIPI CSI interface of lower power consumption. However, when the optical fiber transmission component converts the image data from the electrical signal to the optical signal, there are requirements for a signal amplitude of the image data. For example, when the optical fiber transmission component converts the data from the electrical signal to the optical signal, a minimum signal swing of the data is usually required to be 200mv, while the signal swing of MIPI CSI data is usually less than 200mv. Accordingly, the optical fiber transmission component cannot be supported to convert the MIPI CSI data from the electrical signal to the optical signal, when combined with the losses during the transmission process. Therefore, in this embodiment, the second data communication protocol satisfies the requirement that the image data based on the second data communication protocol has a signal amplitude required by the optical fiber transmission component to convert the image data from the electrical signal to the optical signal.

In an embodiment, since the first data processing device 20 includes a programmable logic gate array device, the second data communication protocol can be a private protocol, which is a protocol standard customized within an enterprise.

In an embodiment, the second data communication protocol is capable of being directly identified by the image processing unit. Usually, if the second data communication protocol is a private protocol, the image data transmitted through the optical fiber transmission component can be directly processed by the image processing unit without requiring protocol conversion. In other words, the optical fiber transmission component 30 can be directly connected with the image processing unit, to output data to the image processing unit for processing without requiring protocol conversion through the data processing device.

Please refer to FIG. 2. In some embodiments, the optical fiber transmission component 30 may include an electro-optic converter 32, an optical fiber transmission channel 39a, and a photo-electric converter 36. The electro-optic converter 32 receives the image data which is outputted by the first data processing device 20, and converts the image data from the electrical signal to the optical signal and outputs said image data to the optical fiber transmission channel 39a.The optical fiber transmission channel 39a transmits the image data which is converted to the optical signal, and in some examples, the optical fiber transmission channel 39a includes an optical fiber. The photo-electric converter 36 receives the image data which is converted to the optical signal and transmitted through the optical fiber transmission channel 39a, and converts the image data from the optical signal to the electrical signal before outputting it to the image processing unit.

In an embodiment, when the image sensor is an eight-channel MIPI interface, the image sensor outputs the image data to the first data processing device through the eight-channel MIPI interface. The first data processing device processes the obtained image data, converts the image data to the image data of one channel, and outputs said image data. Correspondingly, the optical fiber transmission component converts the image data from the electrical signal to the optical signal and transmits it through one optical fiber channel.

In another embodiment, when the image sensor is an eight-channel MIPI interface, the image sensor outputs the image data to the first data processing device through the eight-channel MIPI interface. The first data processing device processes the obtained image data, converts the obtained image data to the image data of two channels, and outputs said image data. Correspondingly, the optical fiber transmission component converts the image data of two channels from the electrical signal to the optical signal, and transmits them through two optical fiber channels.

Usually, when processing the image data by the first data processing device, the number of channels for outputting the image data is smaller than the number of channels for inputting the image data.

One data output channel of the image sensor 10 can correspond to one pin of the image sensor 10 or multiple pins (such as two) of the image sensor 10. Due to the large amount of image data generated by the image sensor, it is difficult to output through one data output channel. Therefore, the generated image data is usually outputted through multiple data output channels to reduce the rate of each output channel. At the same time, by reducing the rate of each output channel, the data processing capacity and power consumption requirement of the next processing unit (such as the first data processing device 20) can be adapted in a large range.

Usually, the image sensor is arranged at the camera head (camera handle). Due to the size and power consumption requirement of the camera head, the subsequent data processing unit, such as the first data processing device, may not have sufficient performance to receive and process the high-speed image data which is outputted by the image sensor, after directly using the MIPI interface image sensor. Therefore, in some embodiments, the image data transmission apparatus may also include one or more bridging circuits, such as a first bridging circuit 41 (FIG. 3) and/or a third bridging circuit 43 (FIG. 4). The main function of the first bridging circuit is to reduce the data transmission rate, that is, the data output channel of the first bridging circuit has a lower data transmission rate than the data output channel of the image sensor, so as to adapt to the subsequent data processing unit, such as the first data processing device.

In an embodiment, the first bridging circuit includes more data output channels than the image sensor.

In an embodiment, the first bridging circuit is configured to convert the image data based on the first data communication protocol to the image data based on a third data communication protocol. The third data communication protocol is different from the first data communication protocol and the second data communication protocol, and the data transmission rate of the third data communication protocol is lower than that of the first data communication protocol. For example, four-channel MIPI format data can be converted to eight-channel Sub-LVDS or LVDS format data and outputted to convert the high-speed MIPI data to the low-speed Sub-LVDS data. LVDS (Low Voltage Differential Signaling) interface, is also known as RS-644 bus interface. LVDS, which is also known as Low Voltage Differential Signal, is a low-power, low bit error rate, low crosstalk, and low radiation differential signal technology that can achieve a transmission rate of over 155Mbps. The core of LVDS technology is the use of extremely low voltage swing for data transmission in a high-speed and differential mode. LVDS was first proposed as a high-speed signal transmission level by National Semiconductor in the United States. Since then, LVDS has been defined in the following two standards: IEEEP1996.3 (passed in March 1996), which mainly targets the Scalable Coherent Interface (SCI) and defines electrical characteristics of LVDS and encoding during packet switching in the SCI protocol; ANS/EIA/EIA-644 (passed in November 1995), which mainly defines electrical characteristics of LVDS and recommends parameters such as maximum transmission rate and theoretical limit rate, etc. The LVDS standard commonly referred to is the latter. The ANS/EIA/EIA-644 standard has been revised and published in 2001. According to the LVDS standard defined in ANS/EIA/EIA-64, the theoretical limit rate is 1.923Gbps. The constant current source mode and low swing output operation mode determine that the IVDS has high-speed driving capability. As the development of LVDS, Sub-LVDS adopts a low swing current mode transmission system. Compared with the traditional voltage mode, when achieving almost the same performance level, Sub-LVDS can work with much lower noise tolerance and much lower swing due to its better resistance ability to power supply noise than the traditional mode. The main challenge in designing an efficient current mode circuit is static power consumption, but this is not a problem in ultra-fast networks, as dynamic power consumption often plays a major role therein. Furthermore, a more advanced process has been adopted here, which reduces the power supply voltage from 2.5 V to 1.8 V and the output voltage swing from 350 mV to 150 mV, thereby achieving lower power consumption and providing higher transmission rate. Sub-LVDS typically supports data transmission of eight to ten channels.

In some embodiments, as shown in FIG. 4, the image data transmission apparatus further includes a third bridging circuit 43 connected between the optical fiber transmission component and the image processing unit. The third bridging circuit 43 is configured to convert the image data based on the second data communication protocol to the image data based on the fourth data communication protocol. The fourth data communication protocol is different from the first data communication protocol and the second data communication protocol, and the data transmission rate of the fourth data communication protocol is lower than that of the second data communication protocol. Usually, due to the fact that the image data processing unit is often located within a camera host, the camera host does not have strict requirements for size and power consumption. Therefore, the main function of the third bridging circuit is to convert the data protocol format to adapt to the protocol format requirement that the image data processing unit accepts, for example, to convert MIPI format data to Sub-LVDS format data.

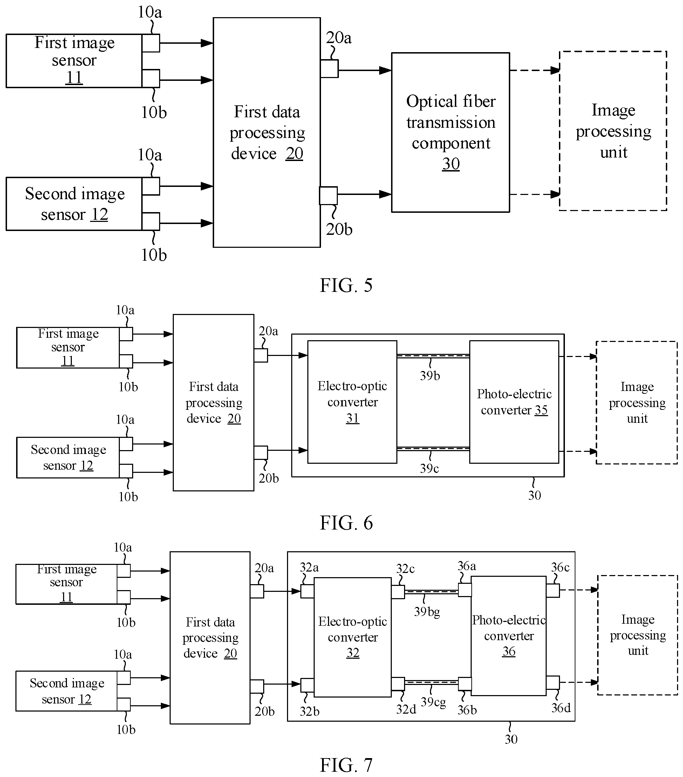

Please refer to FIG. 5. In some embodiments, the image data transmission apparatus includes multiple image sensors, such as a first image sensor 11 and a second image sensor 12. The image data transmission apparatus also includes a first data processing device 20 and an optical fiber transmission component 30, as described in detail below. It should be noted that the image data transmission apparatus shown in the figure, includes an example of two image sensors, but it is not limited to only two image sensors. Actually, N image sensors can be configured according to the requirements, wherein N can be 2 or an integer greater than 2.

The first image sensor 11 and the second image sensor 12 are both configured to generate the image data. The first image sensor and the second image sensor output the image data based on the first data communication protocol through their respective data output channels. In an embodiment, both the first image sensor 11 and the second image sensor 12 include at least two data output channels, such as the data output channel 10a and data output channel 10b. The first image sensor 11 and the second image sensor 12 output the image data through each of the at least two data output channels. For example, the first image sensor 11 outputs the image data through at least two data output channels, such as the data output channel 10a and the data output channel 10b, while the first image sensor 12 outputs the image data through at least two data output channels, such as the data output channel 10a and the data output channel 10b.

The first data processing device 20 includes a programmable logic gate array device, which includes at least a first group of data output terminals 20a and a second group of data output terminals 20b. The first data processing device 20 is configured to convert the image data which is outputted by the first image sensor to a first set of image data based on the second data communication protocol, and output said image data through the first group of data output terminals, as well as convert the image data which is outputted by the second image sensor to a second set of image data based on the second data communication protocol, and output said image data through the second group of data output terminals. The second data communication protocol is different from the first data communication protocol.

It should be noted that the first group of data output terminals 20a and the second group of data output terminals 20b can be either one output terminal or multiple output terminals. Correspondingly, the first set of image data and the second set of image data can be either image data of one channel or image data of multiple channels.

Please refer to FIG. 6. The optical fiber transmission component 30 includes an electro-optic converter 31, a photo-electric converter 35, at least a first optical fiber transmission channel 39b, and a second optical fiber transmission channel 39c. The electro-optic converter 31 converts the first set of image data which is outputted by the first data processing device 20 from the electrical signal to the optical signal, and transmits said image data to the photo-electric converter 35 through the first optical fiber transmission channel 39b. The photo-electric converter 35 then converts the received first set of image data from the optical signal to the electrical signal and outputs said image data. The electro-optic converter 31 also converts the second set of image data which is outputted by the first data processing device 20 from the electrical signal to the optical signal, and transmit said image data to the photo-electric converter 35 through the second optical fiber transmission channel 39c. The photo-electric converter 35 then converts the received second set of image data from the optical signal to the electrical signal and outputs said image data. Among them, the first set of image data and the second set of image data which are outputted by the photo-electric converter 35 are processed by the image processing unit to generate data for image displaying.

In an embodiment, the first optical fiber transmission channel 39b and the second optical fiber transmission channel 39c are two independent signal transmission channels, as explained in detail below.

Please refer to FIG. 7. In some embodiments, the first optical fiber transmission channel 39b includes a first optical fiber 39bg, and the second optical fiber transmission channel 39c includes a second optical fiber 39cg. Specifically, the electro-optic converter 32 includes at least a first input terminal 32a, a second input terminal 32b, a first output terminal 32c, and a second output terminal 32d; while the photo-electric converter 36 includes at least a first input terminal 36a, a second input terminal 36b, a first output terminal 36c, and a second output terminal 36d. The first output terminal 32c of the electro-optic converter32 is connected with the first input terminal 36a of the electro-optic converter36 through the first optical fiber 39bg, the second output terminal 32b of the electro-optic converter 32 is connected with the second input terminal 36b of the photo-electric converter 36 through the second optical fiber 39cg. The first input terminal 32a and the second input terminal 32b of the electro-optic converter 32 are respectively connected with the first group of data output terminals 20a and the second group of data output terminals 20b of the first data processing device 20.

Therefore, the electro-optic converter 32 receives the first set of image data which is outputted by the first data processing device 20 through its first input terminal 32a, and converts the first set of image data from the electrical signal to the optical signal. The electro-optic converter 32 outputs the first set of image data which is converted to the optical signal through its first output terminal 32c, and transmits the first set of image data which is converted to the optical signal through the first optical fiber 39bg. The photo-electric converter 36 receives the first set of image data which is converted to the optical signal and transmitted by the first optical fiber 39bg through its first input terminal 36a, converts the first set of image data from the optical signal to the electrical signal, and outputs the first set of image data through its first output terminal 36c. Similarly, the electro-optic converter 32 receives the second set of image data which is outputted by the first data processing device 20 through its second input terminal 32b, and converts the second set of image data from the electrical signal to the optical signal. The electro-optic converter 32 outputs the second set of image data which is converted to the optical signal through its second output terminal 32b, and transmits said image data through the second optical fiber 39cg. The photo-electric converter 36 receives the second set of image data which is converted to the optical signal and transmitted by the second optical fiber 39cg through its second input terminal 36b, converts the second set of image data from the optical signal to the electrical signal, and outputs the second set of image data through its second output terminal 36d.

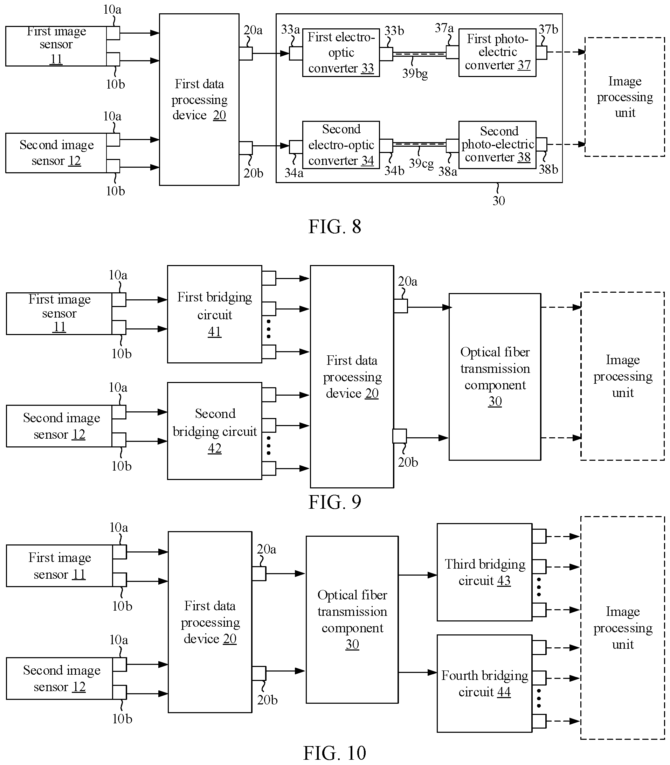

Please refer to FIG. 8. In some embodiments, the electro-optic converter 31 includes a first electro-optic converter 33 and a second electro-optic converter 34, while the photo-electric converter 35 includes a first photo-electric converter 37 and a second photo-electric converter 38. The first optical fiber transmission channel 39b includes a first optical fiber 39bg, and the second optical fiber transmission channel 39c includes a second optical fiber 39cg. Specifically, the first electro-optic converter 33 includes a first input terminal 33a and a first output terminal 33b. The second electro-optic converter 34 includes a second input terminal 34a and a second output terminal 34b. The first photo-electric converter 37 includes a first input terminal 37a and a first output terminal 37b. The second photo-electric converter 38 includes a second input terminal 38a and a second output terminal 38b. The first output terminal 33b of the first electro-optic converter 33 is connected with the first input terminal 37a of the first photo-electric converter 37 through the first optical fiber 39bg, the second output terminal 34a of the second electro-optic converter 34 is connected with the second input terminal 38a of the second photo-electric converter 38 through the second optical fiber 39cg. The first input terminal 33a of the first electro-optic converter 33 is connected with the first group of data output terminals 20a of the first data processing device 20, and the second input terminal 34a of the second electro-optic converter 34 is connected with the second group of data output terminals 20b of the first data processing device 20.

Therefore, the first electro-optic converter 33 receives the first set of image data which is outputted by the first data processing device 20 through its first input terminal 33a, and converts the first set of image data from the electrical signal to the optical signal. The first electro-optic converter 33 outputs the first set of image data which is converted to the optical signal through its first output terminal 33b, and transmits said image data through the first optical fiber 39bg. The first photo-electric converter 37 receives the first set of image data which is converted to the optical signal and transmitted by the first optical fiber 39bg through its first input terminal 37a, converts the first set of image data from the optical signal to the electrical signal, and outputs the first set of image data through its first output terminal 37b. Similarly, the second electro-optic converter 34 receives the second set of image data which is outputted by the first data processing device 20 through its second input terminal 34a, and converts the second set of image data from the electrical signal to the optical signal. The second electro-optic converter 34 outputs the second set of image data which is converted to the optical signal through its second output terminal 34b, and transmits said image data through the second optical fiber 39cg. The second photo-electric converter 38 receives the second set of image data which is converted to the optical signal and transmitted by the second optical fiber 39cg through its second input terminal 38a, converts the second set of image data from the optical signal to the electrical signal, and outputs the second set of image data through its second output terminal 38b.

The above are some explanations of the optical fiber transmission component 30.

In an embodiment, the data output channels of the first and second image sensors are MIPI CSI interfaces, and the first data communication protocol is the MIPI CSI protocol. Specifically, the data output channels of the first and second image sensors can be MIPI CSI-2 interfaces. MIPI is an alliance established in 2003 by companies such as ARM in the UK, Nokia in Finland, ST in Italy, and TI in the US, aiming at standardizing internal interfaces of mobile phones, such as camera interface, display interface, RF/baseband interface, etc., thus reducing the complexity of mobile phone design and increasing design flexibility. There are different working groups under the MIPI Alliance, which have defined a series of internal interface standards for mobile phones, such as camera serial interface (CSI), display serial interface (DSI), RF interface DigRF, microphone/speaker interface SLIMbus, etc. The terminal market requires lower power consumption, higher data transmission rate, and smaller PCB footprint. Among the several standard-based serial differential interfaces currently used, the MIPI interface is suitable for use in the field of power sensitive but high-performance devices. Specifically, as mentioned above, CSI is an interface standard designated by the Camera Working Group of MIPI Alliance, CSI-2 is the second version of MIPI CSI, mainly composed of an application layer, a protocol layer, and a physical layer. It usually supports four-channel data transmission, with a single line transmission speed of up to 1 Gb/s, and also supports eight-channel data transmission. In addition to the ground wire, the MIPI CSI-2 interface generally has one pair of I2C communication pins, one pair of MIPI differential clock pins, and one to four pairs of MIPI differential data signal pins.

Usually, the camera head of the endoscope imaging system is handheld, and the doctor holds the camera head to adjust observed portion and control parameters when performing surgery on a patient. Due to the large amount of data transmitted by the image sensor, the transmission power is high, while significant heat is also generated. The camera head need to be designed with features such as low power consumption and minimal heat generation. The image sensor with MIPI interface has the characteristic of low power consumption, which precisely satisfies the design requirements of the camera head.

Therefore, in order to reduce the power consumption of related devices, this embodiment adopts an image sensor with a MIPI CSI interface of lower power consumption. However, when the optical fiber transmission component converts the image data from the electrical signal to the optical signal, there are requirements for a signal amplitude of the image data. For example, when the optical fiber transmission component converts data from the electrical signal to the optical signal, a minimum signal swing of the data is usually required to be 200mv, while the signal swing of MIPI CSI data is usually less than 200mv. Accordingly, the optical fiber transmission component cannot be supported to convert the MIPI CSI data from the electrical signal to the optical signal, when combined with the losses during the transmission process. Therefore, in this embodiment, the second data communication protocol satisfies the requirement that the image data based on the second data communication protocol has a signal amplitude required by the optical fiber transmission component to convert the image data from the electrical signal to the optical signal.

In an embodiment, since the first data processing device 20 includes a programmable logic gate array device, the second data communication protocol can be a private protocol, which is a protocol standard customized within an enterprise.

In an embodiment, the second data communication protocol is capable of being directly identified by the image processing unit. Usually, if the second data communication protocol is a private protocol, the image data transmitted through the optical fiber transmission component can be directly processed by the image processing unit without requiring protocol conversion.

In some embodiments, the image data transmission apparatus may also include one or more bridging circuits, such as the first bridging circuit 41 and/or the second bridging circuit 42. The function of the bridging circuit is to reduce the data transmission rate, that is, the data output channel of the first bridging circuit has a lower data transmission rate than the data output channel of the first image sensor, and the data output channel of the second bridging circuit has a lower data transmission rate than the data output channel of the second image sensor.

In an embodiment, the first bridging circuit includes more data output channels than the first image sensor, and the second bridging circuit includes more data output channels than the second image sensor.

In an embodiment, the first bridging circuit and the second bridging circuit are configured to convert the image data based on the first data communication protocol to the image data based on the third data communication protocol. The third data communication protocol is different from the first data communication protocol and the second data communication protocol, and the data transmission rate of the third data communication protocol is lower than that of the first data communication protocol.

For example, the first bridging circuit and the second bridging circuit are configured to convert the inputted image data to Sub-LVDS or LVDS format data and output the Sub-LVDS or LVDS format data. LVDS (Low Voltage Differential Signaling) interface, is also known as RS-644 bus interface. LVDS, which is also known as Low Voltage Differential Signal, is a low-power, low bit error rate, low crosstalk, and low radiation differential signal technology that can achieve a transmission rate of over 155Mbps. The core of LVDS technology is the use of extremely low voltage swing for data transmission in a high-speed and differential mode.

FIG. 9 is an example that includes the first bridging circuit 41 and the second bridging circuit 42.

As shown in FIG. 10, In an embodiment, the image data transmission apparatus further includes a third bridging circuit 43 and/or a fourth bridging circuit 44 which are/is connected between the optical fiber transmission component and the image processing unit. The third bridging circuit 43 is configured to convert the first set of image data based on the second data communication protocol to the image data based on the fourth data communication protocol, and the fourth bridging circuit 44 is configured to convert the second set of image data based on the second data communication protocol to the image data based on the fourth data communication protocol. The fourth data communication protocol is different from the first data communication protocol and the second data communication protocol, and the data transmission rate of the fourth data communication protocol is lower than that of the second data communication protocol. In an embodiment, the fourth data communication protocol may be a private protocol.

The image data transmission apparatus provided in the embodiment of this application performs a communication protocol conversion on the image data which is outputted by the image sensor through the first data processing device, which allows for a wider selection range of image sensors during product design, such as the use of image sensors with low-power, high-speed MIPI interfaces.

The image data transmission apparatus in this disclosure can be applied to situations and products such as endoscope imaging systems. Below, we may take the application of the image data transmission apparatus in the endoscope imaging system situation as an example for explanation.

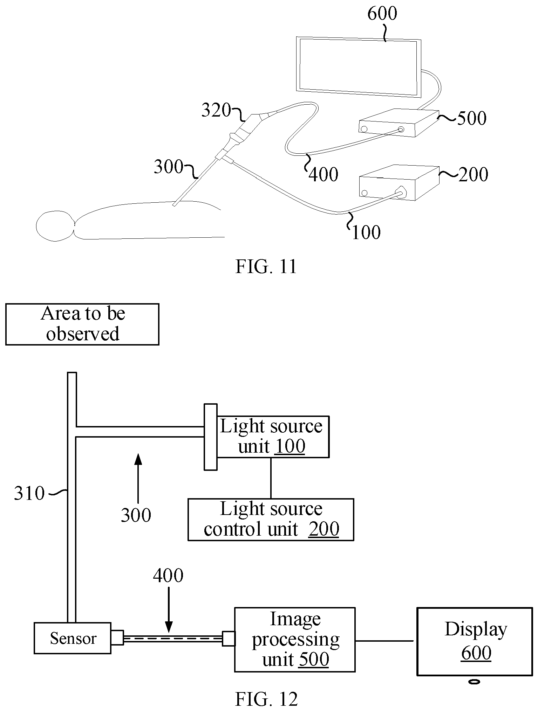

Please refer to FIG. 11, 12, and 13. In some embodiments, the endoscope imaging system includes a light source unit 100, a light source control unit 200, an endoscope 300, a imaging unit 320, an endoscopic data transmission apparatus 400, an image processing unit 500, and a display 600. The detail is explained below.

The light source unit 100 is configured to provide an illumination light source to an area to be observed. The light source unit 100 can provide light required for ordinary light imaging, as well as light required for special light imaging. For example, the light source provided by the light source unit 100 for the area to be observed can be ordinary light illumination based on wideband light and special light illumination based on narrow-band light. The endoscope system generates a color image in the ordinary light mode, or generates a monochromatic image with vascular enhancement effect in the special light mode and then generates a color image based on the grayscale value of the monochromatic image. It can be understood that the color image generated from the monochromatic image, such as a grayscale image, is a pseudo color image, which means that the special light image is a pseudo color image at this time.

In some embodiments, the light source unit 100 may include a first light source 110 and a second light source 120. In normal lighting mode, the first light source 110 can provide multiple monochromatic lights of different wavelength ranges at different times. For example, the first light source 110 can be a semiconductor light source or an LED light source, and the monochromatic light provided can be a blue light, green light, red light, etc. In other embodiments, the first light source 110 can also provide a combination of multiple monochromatic lights or can be a wide spectral white light source. The wavelength range of the monochromatic light is approximately 400nm to 700nm. In special lighting mode, the second light source 120 provides a narrowband light. For example, the second light source 120 can be a laser that emits a narrowband blue laser light, with a peak wavelength of at least one value in the range of 390nm-460nm. In other embodiments, the second light source 120 can also be an LED light source or a laser LED, and the narrowband light emitted can be a narrowband green laser light, etc.

In some embodiments, the light source unit 100 may further include a dichroic mirror 130. Under the control of the light source control unit 200, the first light source 110 and the second light source 120 operate at different time points. That is, when the first light source 110 is turned on, the second light source 120 is turned off, and vice versa. The dichroic mirror 130 is arranged along optical transmission paths of multiple monochromatic lights and narrowband lights, and the optical paths of multiple monochromatic lights and narrowband lights are combined into the same one optical path after passing through the dichroic mirror 130. For example, multiple monochromatic lights can be transmitted through the dichroic mirror 130, and multiple narrowband light can be reflected by the dichroic mirror 130, resulting in the synthesis of the two optical paths into the same one optical path, and vice versa. In the optical path behind the dichroic mirror 130, the narrowband lights and multiple monochromatic lights are transmitted in the direction of the endoscope 300 along the same synthesized optical path at different time points.

In some embodiments, the light source unit 100 also includes a coupling mirror 140 which is arranged at a light source inlet of the dichroic mirror 130 and the endoscope 300. The coupling mirror 140 can focus the light transmitted from the dichroic mirror 130, thereby better guiding it into the endoscope 300, minimizing light loss as much as possible, and improving the overall lighting quality of the system. The optical synthesis effect of the dichroic mirror 130 and the focusing effect of the coupling mirror 140 can better guide the light into the endoscope 300. Meanwhile, the use of dichroic mirror 130 can make the overall structure of the light source unit 100 more compact and the light propagation path shorter.

The above are some explanations of the light source unit 100. The light source control unit 200 is configured to control the light source unit 100, such as to control the light source unit 100 to provide light required for ordinary light imaging and to control the light source unit 100 to provide light required for special light imaging.

The endoscope 300 is used for transmitting optical signals. In some embodiments, the endoscope 300 may include an insertion portion 310. In some embodiments, the insertion portion 310 can be inserted into the interior of the object, for example, the insertion portion 310 is a part of the mirror body, which can be inserted into the interior of the object by the operator. The insertion portion 310 is capable of transmitting the light generated by the light source unit 100 to an induction portion of the part to be observed (which can be a light guiding fiber).

The imaging unit includes at least one sensor for generating image data. For example, in some examples, the imaging unit may include an image sensor 10. For example, in some examples, the imaging unit may include the first image sensor 11 and the second image sensor 12. For example, in some examples, the imaging unit may include N sensors for generating image data, and N may be an integer greater than 2.

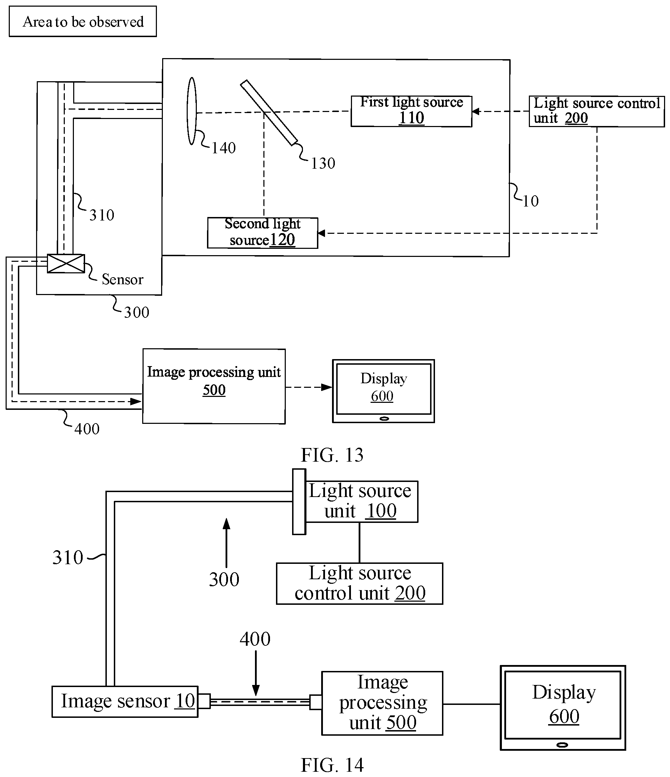

Please refer to FIG. 14 for an example, where the imaging unit includes an image sensor 10. In some examples, the image sensor 10 is configured to generate image data. The image sensor 10 can generate the image data according to specifications that can be processed by the AP (Application Processor) of the CPU for the mobile device.

For example, the image sensor 10 can generate image data of MIPI (Mobile Industry Processor Interface) specification, and correspondingly, the data output channel of the image sensor can be the MIPI CSI-2 interface.

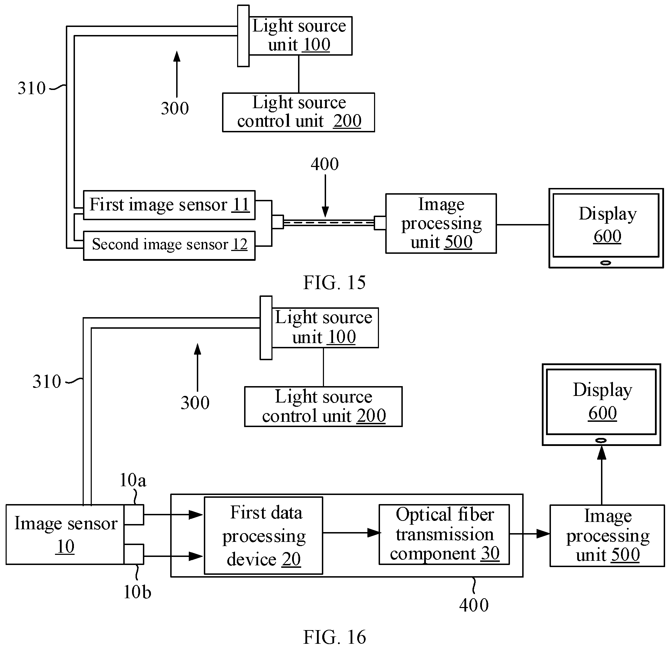

Please refer to FIG. 15 for example, where the imaging unit includes the first image sensor 11 and the second image sensor 12. In some examples, both the first image sensor 11 and the second image sensor 12 are configured to generate the image data.

The first image sensor 11 and the second image sensor 12 can generate the image data according to specifications that can be processed by the AP (Application Processor) of the CPU for a mobile device. For example, the first image sensor 11 and the second image sensor 12 can generate image data of MIPI (Mobile Industry Processor Interface) specification, and correspondingly, the data output channel of the image sensor can be the MIPI CSI-2 interface.

In some examples, one end of the imaging unit is connected with the endoscopic data transmission apparatus 400 to provide image data to the image processing unit 500, and one end of the imaging unit can be clamped at the endoscope. The light source unit 100 provides a light source for the endoscope 300. The imaging unit 320 can then acquire an optical signal of the endoscope. The endoscopic data transmission apparatus 400 is explained in detail as follows.

The endoscopic data transmission apparatus 400 is configured to transmit the image data generated by the sensor in the imaging unit 320 to the subsequent image processing unit 500 for processing. There are various implementation methods for the endoscopic data transmission apparatus 400, as explained in detail below.

Take the example of the imaging unit including the image sensor 10 to illustrate the structure and function of the endoscopic data transmission apparatus 400 in this embodiment. Please refer to FIG. 16. In this case, the endoscopic data transmission apparatus 400 can include a first data processing device 20 and an optical fiber transmission component 30.

The first data processing device 20 includes a programmable logic gate array device for converting the image data which is outputted by the image sensor 10 to the image data based on a second data communication protocol and outputting said image data. Among them, the second data communication protocol is different from the first data communication protocol.

The optical fiber transmission component 30 is configured to convert the image data based on the second data communication protocol, which is outputted by the first data processing device 20, from an electrical signal to an optical signal for transmission, and then convert the optical signal to the electrical signal and output said image data for processing by the image processing unit to generate data for image displaying.

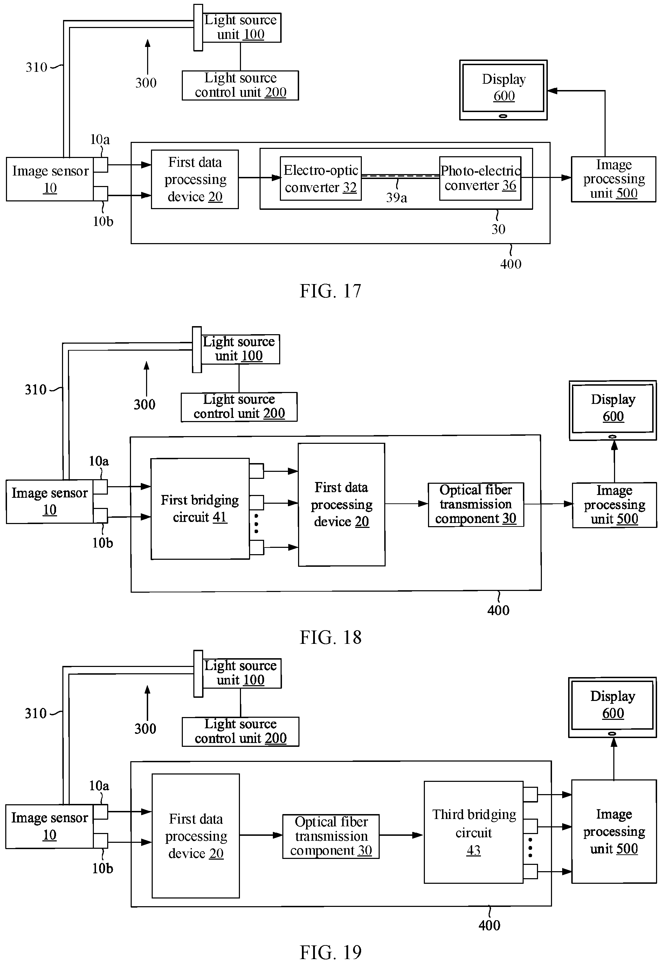

Please refer to FIG. 17. In some embodiments, the optical fiber transmission component 30 may include an electro-optic converter 32, an optical fiber transmission channel 39a, and a photo-electric converter 36. The electro-optic converter 32 receives the image data which is outputted by the first data processing device 20, and converts the image data from the electrical signal to the optical signal and outputs said image data to the optical fiber transmission channel 39a. The optical fiber transmission channel 39a transmits the image data which is converted to the optical signal, and in some examples, the optical fiber transmission channel 39a includes an optical fiber. The photo-electric converter 36 receives the image data which is converted to the optical signal and transmitted through the optical fiber transmission channel 39a, and converts the image data from the optical signal to the electrical signal before outputting said image data.

In some embodiments, the endoscopic data transmission apparatus 400 may also include one or more bridging circuits, such as a first bridging circuit 41 (FIG. 18) and/or a third bridging circuit 43 (FIG. 19). The function of the bridging circuit is to reduce the data transmission rate to adapt to subsequent data processing units, such as the first data processing device.

In an embodiment, the first bridging circuit includes more data output channels than the image sensor.

In an embodiment, the first bridging circuit is configured to convert the image data based on the first data communication protocol to the image data based on the third data communication protocol. The third data communication protocol is different from the first data communication protocol and the second data communication protocol, and the data transmission rate of the third data communication protocol is lower than that of the first data communication protocol.

The third bridging circuit 43 is configured to convert the image data based on the second data communication protocol to the image data based on a fourth data communication protocol. The fourth data communication protocol is different from the first data communication protocol and the second data communication protocol, and the data transmission rate of the fourth data communication protocol is lower than that of the second data communication protocol.

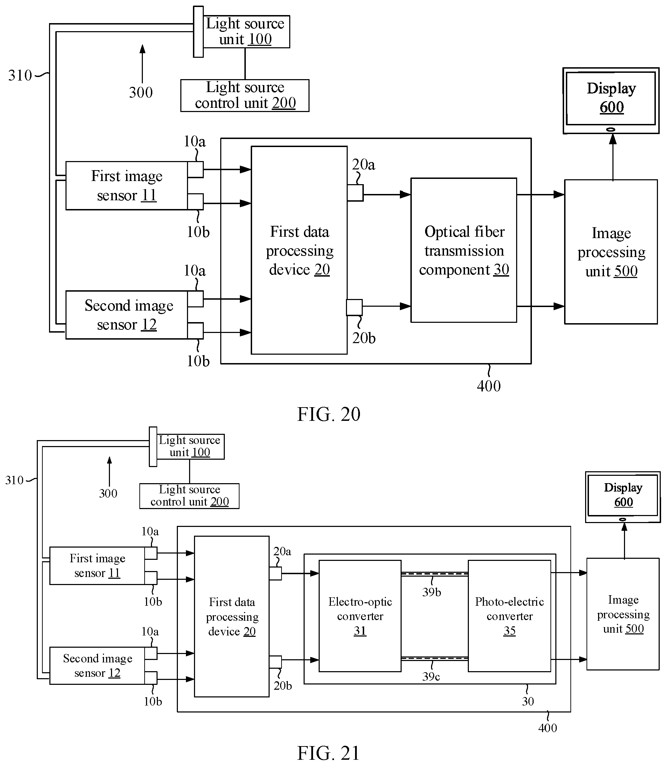

Take another example of the imaging unit including the first image sensor 11 and the second image sensor 12 to illustrate the structure and function of the endoscopic data transmission apparatus 400 in this embodiment. Please refer to FIG. 20. In this case, the endoscopic data transmission apparatus 400 can include a first data processing device 20 and an optical fiber transmission component 30.

The first image sensor 11 and the second image sensor 12 are both configured to generate image data. The first image sensor and the second image sensor output the image data based on the first data communication protocol through their respective data output channels.

The first data processing device 20 includes a programmable logic gate array device, which includes at least a first group of data output terminals 20a and a second group of data output terminals 20b. The first data processing device 20 is configured to convert the image data which is outputted by the first image sensor to a first set of image data based on the second data communication protocol, and output it through the first group of data output terminal, as well as convert the image data which is outputted by the second image sensor to a second set of image data based on the second data communication protocol, and output said image data through the second group of data output terminal. The second data communication protocol is different from the first data communication protocol.

Please refer to FIG. 21, where the optical fiber transmission component 30 includes an electro-optic converter 31, a photo-electric converter 35, at least a first optical fiber transmission channel 39b, and a second optical fiber transmission channel 39c. The electro-optic converter 31 converts the first set of image data which is outputted by the first data processing device 20, from the electrical signal to the optical signal, and transmit said image data to the photo-electric converter 35 through the first optical fiber transmission channel 39b. The photo-electric converter 35 then converts the received first set of image data from the optical signal to the electrical signal and outputs said image data. The electro-optic converter 31 further converts the second set of image data which is outputted by the first data processing device 20 from the electrical signal to the optical signal, and transmit said image data to the photo-electric converter 35 through the second optical fiber transmission channel 39c. The photo-electric converter 35 then converts the received second set of image data from the optical signal to the electrical signal and outputs said image data. Among them, the first set of image data and the second set of image data which are outputted by the photo-electric converter 35 are processed by the image processing unit to generate data for image displaying.

In an embodiment, the first optical fiber transmission channel 39b and the second optical fiber transmission channel 39c are two independent signal transmission channels, as explained in detail below.

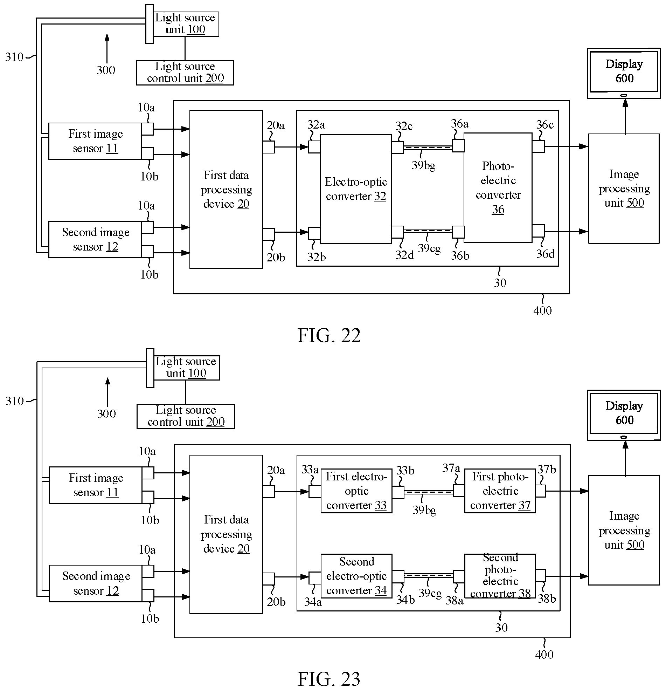

Please refer to FIG. 22. In some embodiments, the first optical fiber transmission channel 39b includes a first optical fiber 39bg, and the second optical fiber transmission channel 39c includes a second optical fiber 39cg. Specifically, the electro-optic converter 32 includes at least a first input terminal 32a, a second input terminal 32b, a first output terminal 32c, and a second output terminal 32d. The photo-electric converter 36 includes at least a first input terminal 36a, a second input terminal 36b, a first output terminal 36c, and a second output terminal 36d. The first output terminal 32c of the electro-optic converter 32 is connected with the first input terminal 36a of the photo-electric converter 36 through the first optical fiber 39bg. The second output terminal 32b of the electro-optic converter 32 is connected with the second input terminal 36b of the photo-electric converter 36 through the second optical fiber 39cg. The first input terminal 32a and the second input terminal 32b of the electro-optic converter 32 are respectively connected with the first group of data output terminals 20a and the second group of data output terminals 20b of the first data processing device 20.

Therefore, the electro-optic converter 32 receives the first set of image data which is outputted by the first data processing device 20 through its first input terminal 32a, and converts the first set of image data from the electrical signal to the optical signal. The electro-optic converter 32 outputs the first set of image data which is converted to the optical signal through its first output terminal 32c, and transmits said image data through the first optical fiber 39bg. The photo-electric converter 36 receives the first set of image data which is converted to the optical signal and transmitted by the first optical fiber 39bg through its first input terminal 36a, converts the first set of image data from the optical signal to the electrical signal, and outputs the first set of image data through its first output terminal 36c. Similarly, the electro-optic converter 32 receives the second set of image data which is outputted by the first data processing device 20 through its second input terminal 32b, and converts the second set of image data from the electrical signal to the optical signal. The electro-optic converter 32 outputs the second set of image data which is converted to the optical signal through its second output terminal 32b, and transmits it through the second optical fiber 39cg. The photo-electric converter 36 receives the second set of image data which is converted to the optical signal and transmitted by the second optical fiber 39cg through its second input terminal 36b, converts the second set of image data from the optical signal to the electrical signal, and outputs the second set of image data through its second output terminal 36d.

Please refer to FIG. 23. In some embodiments, the electro-optic converter 31 includes a first electro-optic converter 33 and a second electro-optic converter 34. The photo-electric converter 35 includes a first photo-electric converter 37 and a second photo-electric converter 38. The first optical fiber transmission channel 39b includes a first optical fiber 39bg, and the second optical fiber transmission channel 39c includes a second optical fiber 39cg. Specifically, the first electro-optic converter 33 includes a first input terminal 33a and a first output terminal 33b. The second electro-optic converter 34 includes a second input terminal 34a and a second output terminal 34b. The first photo-electric converter 37 includes a first input terminal 37a and a first output terminal 37b. The second photo-electric converter 38 includes a second input terminal 38a and a second output terminal 38b. The first output terminal 33b of the first electro-optic converter 33 is connected with the first input terminal 37a of the first photo-electric converter 37 through the first optical fiber 39bg. The second output terminal 34a of the second electro-optic converter 34 is connected with the second input terminal 38a of the second photo-electric converter 38 through the second optical fiber 39cg. The first input terminal 33a of the first electro-optic converter 33 is connected with the first group of data output terminals 20a of the first data processing device 20, and the second input terminal 34a of the second electro-optic converter 34 is connected with the second group of data output terminals 20b of the first data processing device 20.

Therefore, the first electro-optic converter 33 receives the first set of image data which is outputted by the first data processing device 20 through its first input terminal 33a, and converts the first set of image data from the electrical signal to the optical signal. The first electro-optic converter 33 outputs the first set of image data which is converted to the optical signal through its first output terminal 33b, and transmits it through the first optical fiber 39bg. The first photo-electric converter 37 receives the first set of image data which is converted to the optical signal and transmitted by the first optical fiber 39bg through its first input terminal 37a, converts the first set of image data from the optical signal to the electrical signal, and outputs the first set of image data through its first output terminal 37b. Similarly, the second electro-optic converter 34 receives the second set of image data which is outputted by the first data processing device 20 through its second input terminal 34a, and converts the second set of image data from the electrical signal to the optical signal. The second electro-optic converter 34 outputs the second set of image data which is converted to the optical signal through its second output terminal 34b, and transmits it through the second optical fiber 39cg. The second photo-electric converter 38 receives the second set of image data which is converted to the optical signal and transmitted by the second optical fiber 39cg through its second input terminal 38a, converts the second set of image data from the optical signal to the electrical signal and outputs the second set of image data through its second output terminal 38b.

The above are some explanations of optical fiber transmission component 30.

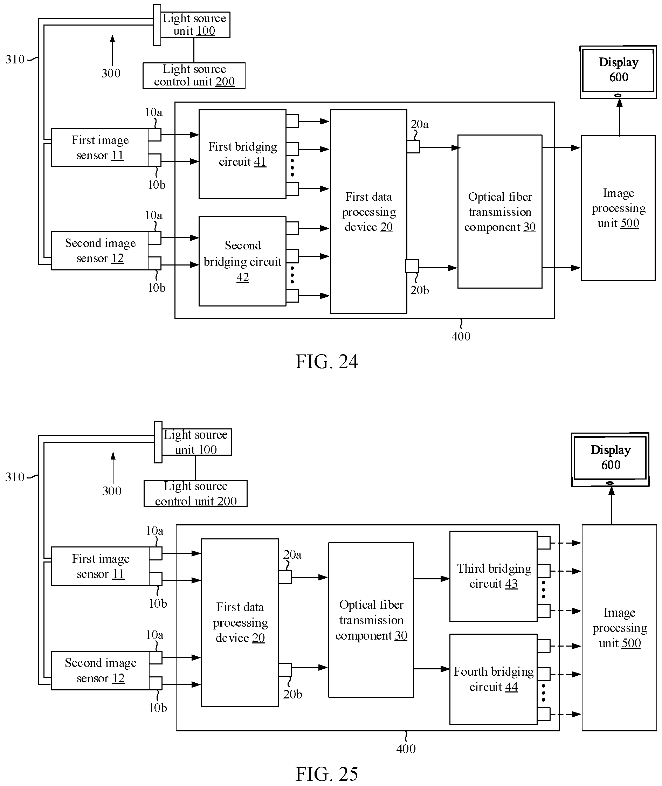

As shown in FIG. 24, in some embodiments, the endoscopic data transmission apparatus 400 may also include one or more bridging circuits, such as the first bridging circuit 41 and/or the second bridging circuit 42. The function of the bridging circuit is to reduce the data transmission rate, that is, the data output channel of the first bridging circuit has a lower data transmission rate than the data output channel of the first image sensor, and the data output channel of the second bridging circuit has a lower data transmission rate than the data output channel of the second image sensor.

In an embodiment, the first bridging circuit includes more data output channels than the first image sensor, and the second bridging circuit includes more data output channels than the second image sensor.

In an embodiment, the first bridging circuit and the second bridging circuit are configured to convert the image data based on the first data communication protocol to the image data based on the third data communication protocol. The third data communication protocol is different from the first data communication protocol and the second data communication protocol, and the data transmission rate of the third data communication protocol is lower than that of the first data communication protocol.

For example, the first bridging circuit and the second bridging circuit are configured to convert the inputted image data to Sub-LVDS or LVDS format data and output said Sub-LVDS or LVDS format data. LVDS (Low Voltage Differential Signaling) interface, is also known as RS-644 bus interface. LVDS, which is also known as Low Voltage Differential Signal, is a low-power, low bit error rate, low crosstalk, and low radiation differential signal technology that can achieve a transmission rate of over 155Mbps. The core of LVDS technology is the use of extremely low voltage swing for data transmission in a high-speed and differential mode.

As shown in FIG. 25, in an embodiment, the endoscopic data transmission apparatus 400 further includes a third bridging circuit 43 and/or a fourth bridging circuit 44 which are/is connected between the optical fiber transmission component and the image processing unit. The third bridging circuit 43 is configured to convert the first set of image data based on the second data communication protocol to the image data based on the fourth data communication protocol, and the fourth bridging circuit 44 is configured to convert the second set of image data based on the second data communication protocol to the image data based on the fourth data communication protocol. The fourth data communication protocol is different from the first data communication protocol and the second data communication protocol, and the data transmission rate of the fourth data communication protocol is lower than that of the second data communication protocol. In an embodiment, the fourth data communication protocol may be a private protocol.

The endoscope imaging system provided in the implementation example of this application performs a communication protocol conversion on the image data which is outputted by the image sensor through the first data processing device, which allows for a wider selection of image sensors during product design, such as the use of low-power, high-speed MIPI interface image sensors.

The above are some explanations of the endoscopic data transmission apparatus 400. In other embodiments, the imaging unit 320 may also include more than three image sensors, and the transmission principle of image data can refer to two image sensors (the first image sensor 11 and the second image sensor 12).

The image processing unit 500 can serve as the image processing host of the endoscope imaging system, and receive and process the image data which is outputted by the endoscopic data transmission apparatus 400 to generate the data for image displaying.

Display 600 is configured to display the data for image displaying.

The above is an explanation of the endoscope imaging system in some embodiments of this disclosure. Skilled technicians should understand that FIGS 11 to 25 are only examples of endoscope imaging systems and do not constitute a limitation of endoscope imaging systems. Endoscope imaging systems may include more or fewer components than shown in FIGS 11 to 25, or combinations of certain components, or different components. For example, the endoscope imaging system may also include expanders, smoke control devices, input/output devices, network access devices, etc. In addition, components with the same reference number as FIGS. 11-25 and 1-10 can be the same, and specific descriptions can be found in the corresponding embodiments of FIGS. 1-10.

This disclosure refers to various exemplary embodiments for explanation. However, those skilled in this field can recognize that changes and modifications can be made to exemplary embodiments without departing from the scope of this disclosure. For example, various operational steps and components which are configured to execute these operational steps can be implemented in different ways based on specific applications or considering any number of cost functions associated with system operations (for example, one or more steps can be deleted, modified, or combined into other steps).

Although the principles of this disclosure have been demonstrated in various embodiments, many modifications of structures, arrangements, proportions, elements, materials, and components that are particularly suitable for specific environments and operational requirements can be used without departing from the principles and scope of this disclosure. The above modifications and other changes or revisions are included within the scope of this disclosure.

The above specific description has been described with reference to various embodiments. However, those skilled in the art recognize that various modifications and changes can be made without departing from the scope of this disclosure. Therefore, the consideration of this disclosure is explanatory rather than restrictive, and all these modifications are included within its scope. Similarly, there are solutions for the advantages, other advantages, and problems of various embodiments as described above. However, the benefits, advantages, solutions to problems, and any solutions that can generate or make them clearer should not be interpreted as critical, necessary, or essential. The term "including" and any other variations thereof used in this disclosure belong to non-exclusive inclusion, which include not only these elements but also other elements that are not explicitly listed or do not belong to the process, method, system, article, or device. In addition, the term "coupling" and any other variations thereof used in this disclosure refer to physical connections, electrical connections, magnetic connections, optical connections, communication connections, functional connections, and/or any other connections.

Those skilled in the art can recognize that many changes can be made to the details of the aforementioned embodiments without departing from the basic principles of this disclosure. Therefore, the scope of this disclosure should be determined solely by the claims.

## Claims

1. An image data transmission apparatus for an endoscope imaging system, wherein the image data transmission apparatus is connected with an image processing unit in the endoscope imaging system and is configured to transmit image data to the image processing unit;
**characterized in that**, the image data transmission apparatus comprises:
at least a first image sensor and a second image sensor; wherein the first image sensor and the second image sensor are both configured to generate image data, the first image sensor and the second image sensor are further configured to output the image data based on a first data communication protocol through their respective data output channel;
a first data processing device which comprises a programmable logic gate array device; wherein the programmable logic gate array device comprises at least a first group of data output terminals and a second group of data output terminals; wherein the first data processing device is configured to convert the image data which is outputted by the first image sensor to a first set of image data based on a second data communication protocol, and output the first set of image data through the first group of data output terminals, as well as to convert the image data which is outputted by the second image sensor to a second set of image data based on the second data communication protocol, and output the second set of image data through the second group of data output terminals; wherein the second data communication protocol is different from the first data communication protocol;
an optical fiber transmission component, which comprises an electro-optic converter, a photo-electric converter, at least a first optical fiber transmission channel and a second optical fiber transmission channel; wherein the electro-optic converter is configured to convert the first set of image data which is outputted by the first data processing device from an electrical signal to an optical signal, and transmit the converted first set of image data to the photo-electric converter through the first optical fiber transmission channel; and the photo-electric converter is configured to convert the received first set of image data from the optical signal to an electrical signal and output the further converted first set of image data; wherein the electro-optic converter is further configured to convert the second set of image data which is outputted by the first data processing device from an electrical signal to an optical signal, and transmit the converted second set of image data to the photo-electric converter through the second optical fiber transmission channel; and the photo-electric converter is further configured to convert the received second set of image data from the optical signal to an electrical signal and output the further converted second set of image data; wherein the further converted first set of image data and the further converted second set of image data, which are outputted by the photo-electric converter, are processed by the image processing unit to generate data for image displaying.

2. The image data transmission apparatus according to claim 1, **characterized in that**, the respective data output channel of the first image sensor and the second image sensor is an MIPI CSI interface, and the first data communication protocol is an MIPI CSI protocol.

3. The image data transmission apparatus according to claim 2, **characterized in that**, the second data communication protocol satisfies a requirement that the image data based on the second data communication protocol have a signal amplitude which is required for converting the image data from the electrical signal to the optical signal by the electro-optic converter.

4. The image data transmission apparatus according to any one of claims 1-3, **characterized in that**, the second data communication protocol is capable of being directly identified by the image processing unit.

5. The image data transmission apparatus according to any one of claims 1-3, **characterized in that**, the image data transmission apparatus further comprises a first bridging circuit which is connected between the first image sensor and the first data processing device, and/or a second bridging circuit which is connected between the second image sensor and the first data processing device.

6. The image data transmission apparatus according to claim 5, **characterized in that**, a data output channel of the first bridging circuit has a lower data transmission rate than the data output channel of the first image sensor, and a data output channel of the second bridging circuit has a lower data transmission rate than the data output channel of the second image sensor.

7. The image data transmission apparatus according to claim 5, **characterized in that**, the first bridging circuit comprises more data output channels than the first image sensor, and the second bridging circuit comprises more data output channels than the second image sensor.

8. The image data transmission apparatus according to claim 5, **characterized in that**, the first bridging circuit and the second bridging circuit are configured to convert the image data based on the first data communication protocol to image data based on a third data communication protocol; wherein the third data communication protocol is different from the first data communication protocol and the second data communication protocol, and the third data communication protocol has a lower data transmission rate than the first data communication protocol.

9. The image data transmission apparatus according to any one of claims 1-3, 5-8, **characterized in that**, the image data transmission apparatus further comprises a third bridging circuit and/or a fourth bridging circuit which are/is connected between the optical fiber transmission component and the image processing unit; wherein the third bridging circuit is configured to convert the first set of image data based on the second data communication protocol to image data based on a fourth data communication protocol, and the fourth bridging circuit is configured to convert the second set of image data based on the second data communication protocol to image data based on the fourth data communication protocol; wherein the fourth data communication protocol is different from the first data communication protocol and the second data communication protocol, and the fourth data communication protocol has a lower data transmission rate than the second data communication protocol.

10. The image data transmission apparatus according to claim 1, **characterized in that**, the first optical fiber transmission channel comprises a first optical fiber, and the second optical fiber transmission channel comprises a second optical fiber;
the electro-optic converter comprises at least a first input terminal, a second input terminal, a first output terminal, and a second output terminal; the photo-electric converter comprises at least a first input terminal, a second input terminal, a first output terminal, and a second output terminal; wherein the first output terminal of the electro-optic converter is connected with the first input terminal of the photo-electric converter through the first optical fiber; the second output terminal of the electro-optic converter is connected with the second input terminal of the photo-electric converter through the second optical fiber;
the electro-optic converter is further configured to receive the first set of image data which is outputted by the first data processing device through the first input terminal of the electro-optic converter, and to convert the first set of image data from the electrical signal to the optical signal; the electro-optic converter is further configured to output the first set of image data, which is converted to the optical signal, through the first output terminal of the electro-optic converter, and to transmit the converted first set of image data through the first optical fiber; the photo-electric converter is further configured to receive the first set of image data which is converted to the optical signal and transmitted by the first optical fiber through the first input terminal of the photo-electric converter, to convert the converted first set of image data from the optical signal to the electrical signal, and to output the further converted first set of image data through the first output terminal of the photo-electric converter;
the electro-optic converter is further configured to receive the second set of image data which is outputted by the first data processing device through the second input terminal of the electro-optic converter, and to convert the second set of image data from the electrical signal to the optical signal; the electro-optic converter is further configured to output the second set of image data, which is converted to the optical signal, through the second output terminal of the electro-optic converter, and to transmit the converted second set of image data through the second optical fiber; the photo-electric converter is further configured to receive the second set of image data which is converted to the optical signal and transmitted by the second optical fiber through the second input terminal of the photo-electric converter, to convert the converted second set of image data from the optical signal to the electrical signal, and to output the further converted second set of image data through the second output terminal of the photo-electric converter.

11. The image data transmission apparatus according to claim 1, **characterized in that**, the electro-optic converter comprises a first electro-optic converter and a second electro-optic converter; the photo-electric converter comprises a first photo-electric converter and a second photo-electric converter; wherein the first optical fiber transmission channel comprises a first optical fiber, and the second optical fiber transmission channel comprises a second optical fiber;
the first electro-optic converter comprises a first input terminal and a first output terminal, the second electro-optic converter comprises a second input terminal and a second output terminal, the first photo-electric converter comprises a first input terminal and a first output terminal, the second photo-electric converter comprises a second input terminal and a second output terminal; wherein the first output terminal of the first electro-optic converter is connected with the first input terminal of the first photo-electric converter through the first optical fiber; the second output terminal of the second electro-optic converter is connected with the second input terminal of the second photo-electric converter through the second optical fiber;
the first electro-optic converter is configured to receive the first set of image data which is outputted by the first data processing device through the first input terminal of the first electro-optic converter, and to convert the first set of image data from the electrical signal to the optical signal; the first electro-optic converter is further configured to output the first set of image data which is converted to the optical signal through the first output terminal of the first electro-optic converter, and to transmit the converted first set of image data through the first optical fiber; the first photo-electric converter is configured to receive the first set of image data which is converted to the optical signal and transmitted by the first optical fiber through the first input terminal of the first photo-electric converter, to convert the converted first set of image data from the optical signal to the electrical signal, and to output the further converted first set of image data through the first output terminal of the first photo-electric converter;
the second electro-optic converter is configured to receive the second set of image data which is outputted by the first data processing device through the second input terminal of the second electro-optic converter, and to convert the second set of image data from the electrical signal to the optical signal; the second electro-optic converter is further configured to output the second set of image data which is converted to the optical signal through the second output terminal of the second electro-optic converter, and to transmit the converted second set of image data through the second optical fiber; the second photo-electric converter is configured to receive the second set of image data which is converted to the optical signal and transmitted by the second optical fiber through the second input terminal of the second photo-electric converter, to convert the converted second set of image data from the optical signal to the electrical signal, and to output the further converted second set of image data through the second output terminal of the second photo-electric converter.

12. An image data transmission apparatus for an endoscope imaging system, wherein the image data transmission apparatus is connected with an image processing unit in the endoscope imaging system and is configured to transmit image data to the image processing unit;
**characterized in that**, the image data transmission apparatus comprises:
an image sensor which is configured to generate and output image data based on a first data communication protocol;
a first data processing device which comprises a programmable logic gate array device; wherein the first data processing device is configured to at least convert the image data, which is outputted by the image sensor, to image data based on a second data communication protocol, and to output the image data based on the second data communication protocol; wherein the second data communication protocol is different from the first data communication protocol;
an optical fiber transmission component, which is configured to convert the image data based on the second data communication protocol, which data is outputted by the first data processing device, from an electrical signal to an optical signal; and to transmit the image data which is converted to the optical signal; and then to convert the image data, which is converted to the optical signal, to an electrical signal; and to output the image data which is converted to the electrical signal to the image processing unit for processing, so as to generate data for image displaying.

13. The image data transmission apparatus according to claim 12, **characterized in that**, a data output channel of the image sensor is an MIPI CSI interface, and the first data communication protocol is an MIPI CSI protocol.

14. The image data transmission apparatus according to claim 13, **characterized in that**, the second data communication protocol satisfies a requirement that the image data based on the second data communication protocol have a signal amplitude which is required for converting the image data from the electrical signal to the optical signal by the optical fiber transmission component.

15. The image data transmission apparatus according to any one of claims 12-14, **characterized in that**, the second data communication protocol is capable of being directly identified by the image processing unit.

16. The image data transmission apparatus according to any one of claims 12-14, **characterized in that**, the image data transmission apparatus further comprises a first bridging circuit which is connected between the image sensor and the first data processing device.

17. The image data transmission apparatus according to claim 16, **characterized in that**, a data output channel of the first bridging circuit has a lower data transmission rate than a data output channel of the image sensor.

18. The image data transmission apparatus according to claim 16, **characterized in that**, the first bridging circuit comprises more data output channels than the image sensor.

19. The image data transmission apparatus according to claim 16, **characterized in that**, the first bridging circuit is configured to convert the image data based on the first data communication protocol to the image data based on a third data communication protocol; wherein the third data communication protocol is different from the first data communication protocol and the second data communication protocol; and the third data communication protocol has a lower data transmission rate than the first data communication protocol.

20. The image data transmission apparatus according to any one of claims 12-14, 16-19, **characterized in that**, the image data transmission apparatus further comprises a third bridging circuit which is connected between the optical fiber transmission component and the image processing unit, wherein the third bridging circuit is configured to convert the image data based on the second data communication protocol to image data based on a fourth data communication protocol; wherein the fourth data communication protocol is different from the first data communication protocol and the second data communication protocol, and the fourth data communication protocol has a lower data transmission rate than the second data communication protocol.

21. The image data transmission apparatus according to any one of claims 12-20, **characterized in that**, the optical fiber transmission component comprises an electro-optic converter, an optical fiber transmission channel, and a photo-electric converter;
the electro-optic converter is configured to receive the image data which is outputted by the first data processing device, and to convert the image data from the electrical signal to the optical signal for outputting to the optical fiber transmission channel;
the optical fiber transmission channel is configured to transmit the image data which is converted to the optical signal, wherein the optical fiber transmission channel comprises an optical fiber;
the photo-electric converter is configured to receive the image data which is converted to the optical signal and transmitted through the optical fiber transmission channel, to convert the converted image data from the optical signal to the electrical signal, and to output the image data which is further converted to the electrical signal.

22. An endoscope imaging system, **characterized in that**, comprising
a light source unit;
a light source control unit, which is configured to control the light source unit to provide imaging light;
an endoscope, which comprises an insertion portion which is insertable into an interior of an object;
an imaging unit, which comprises the image data transmission apparatus according to any one of claims 1-21;
the image processing unit, which is configured to receive and analyze the image data, which is outputted by the image data transmission apparatus, to generate the data for image displaying; and
a display, which is configured to display the data for image displaying.

23. The endoscope imaging system according to claim 22, **characterized in that**, the image processing unit comprises the programmable logic gate array or a central processor.
